# EUROPEAN PATENT SPECIFICATION

(11) **EP 1 882 929 B1**
(45) Date of publication and mention of the grant of the patent: **12.10.2011**
(21) Application number: 06291226.6
(22) Date of filing: 28.07.2006
(51) Int. Cl.: G01N 23/204, G01N 23/222, G01V 5/00

(54) **A detection system and detection method based on pulsed energetic particles**
Detektionssystem und Detektionsverfahren auf Basis von gepulsten energetischen Teilchen
Système de détection et procédé de détection basé sur les particules énergétiques pulsées

(43) Date of publication of application: 30.01.2008
(73) Proprietor: Sage Innovations, Inc., Port Louis (MU)
(72) Inventor: Peter, Choi, 91400 Orsay (FR)
(74) Representative: Le Forestier, Eric

(56) References cited:
- WO-A-91/11010
- WO-A-99/53344
- DE-A1- 10 323 093
- US-A- 5 200 626
- US-A- 5 446 288
- VOURVOPOULOS G ET AL: "A PULSED FAST-THERMAL NEUTRON SYSTEM FOR THE DETECTION OF HIDDEN EXPLOSIVES" NUCLEAR INSTRUMENTS & METHODS IN PHYSICS RESEARCH, SECTION - B: BEAM INTERACTIONS WITH MATERIALS AND ATOMS, ELSEVIER, AMSTERDAM, NL, vol. B79, no. 1/4, 2 June 1993 (1993-06-02), pages 585-588, XP000381502 ISSN: 0168-583X

## Description

### Field of the invention

The invention relates to active neutron interrogation technology and more particularly to a detection system that uses neutrons to interrogate and detect materials or compounds in items.

### Background of the invention

So-called 'neutronic interrogation' is the only non-invasive technology known to date which allow to extract, from a shielded and sealed item, a signature that is related to the chemical elements or compounds contained in the item.

Such technology therefore brings significant progress over X-ray machines and the like which allow to discriminate between items only by their shape and density of material, and is used for various applications, e.g. the detection of explosives, nuclear materials or contrabands such as narcotics in items or buildings.

A number of neutron-based detection techniques have been developed, depending on the practical application.

Thermal Neutron Analysis (TNA) has been tried, for example, for inspection of checked baggage at airports. More specifically, low-energy neutrons cause nitrogen contained in certain explosives to emit gamma rays and cause fissile materials to give off neutrons of their own. However, the first-generation TNA screeners produced an unacceptable rate of false alarms, due to a large number of nitrogen-bearing articles contained in a typical baggage. Furthermore, TNA requires moderators to slow down fast neutrons from a source to thermal neutrons.

Pulsed Fast Thermal Neutron Analysis (PFTNA) of baggage for contraband such as explosives and narcotics has also been proposed. It combines the detection of gamma ray emissions from several different neutron interactions in a single system and used a short pulse high-energy neutron to perform FNA (Fast Neutron Analysis) interrogation. A suitable neutron generator is typically described in US 5 200 626. This makes possible to separate in time the FNA and TNA interrogations and improves the quality and statistics of the gamma signatures measured.

Typically, a single repetitively pulsed neutron generator based on the deuterium-tritium reaction is used to produce a short pulse (several µs) of 14 MeV neutrons. During this pulse, the interaction is primarily due to the inelastic scattering of high energy neutrons in the object being interrogated. The gamma ray emission is primarily composed of prompt gamma photons from (n. n'y) and (n. py) reactions. The pulse is repeated with a frequency of 10 kHz and the spectra of the prompt gamma photons from the high energy neutron interaction are collected using conventional single photon counting gamma spectroscopy technique. From these spectra, gamma signatures from elements such as C and O can be extracted.

In between the neutron pulses, part of the fast neutrons continue to collide with the background material, in particular the light elements, and slow down to thermal energy.

When a neutron has energy below 1 eV, it can easily be captured by elements such as H, N or Fe, producing prompt gamma photons from such (n. y) capture reactions. A separate thermal neutron reaction spectrum is constructed from the gamma emission detected during this period.

After a predetermined number of pulses, there is a long pause which allows the detection of gamma photons emitted from elements such as O, Al and Si which has been activated and decay with a delayed gamma reaction.

By cycling through these three steps, three separate spectra are generated, representing the fast neutron interaction, the thermal neutron interaction and the delayed activation interaction. These three spectra would allow, in principle, a clear determination of all the four basic elements in high explosives.

In practice, the identification of a substance in PFTNA is performed by examining the atomic ratios, e.g. the ratio of carbon atoms to oxygen atoms (C/O). This is done by taking the ratio of the intensities for carbon and oxygen gamma rays and then applying the ratio of the cross-sections for the gamma inducing reactions of these elements.

Unlike the FNA process with inherently mixed gamma signals from the TNA process, the PFTNA process specifically separates the spectra due to the FNA and the TNA processes, which improves the identification of the elements of interest in explosive detection.

The short and specific time during which the gamma rays from FNA process are emitted and collected improves the signal to background noise ratio of the gamma signatures from C and O compared with conventional FNA technique.

This improvement, however, is significant only if there are very few thermal neutron capture reactions during the time of FNA process. However, in the context of concealed explosives, there is often a large region, besides the small region occupied by the explosives, where a signal from thermal neutron capture reactions will be emitted. This will significantly deteriorate the signal to noise ratio, which in turn means that a long interrogation time is again necessary to obtain useful statistics.

Furthermore, in the context of luggage inspection, the presence of large quantity of plastic materials from the outer casing to the content, will strongly affect the identification of explosives made from a measurement of the C/O or N/O ratio.

Another known interrogation technique is PFNA (Pulsed Fast Neutron Analysis) and is based on the velocity/energy relationship of a fast neutron, whereby a very short and high energy neutron pulse of a few nanoseconds duration can provide an opportunity to record the spatial region of the neutron interaction, from the time of flight (TOF) of the neutron through the object under interrogation.

This technique is for example described in the document WO 91/11010.

In order to collect the TOF information, a mono-energetic fast neutron pulse is used, so that the spatial position of the neutron at any given time can be calculated. When a fast neutron makes a gamma emitting collision with an element in the object under interrogation, it is then possible to associate the time of detection (and therefore emission) of this gamma photon with a given position in the object. By using a repetitively pulsed neutron source of such very short duration, and by measuring the time of occurrence relative to the launch time of the neutron pulse, as well as the energy of the gamma emission, the elemental density of a range of elements inside a bulk material, along the path of the fast neutron, can be determined.

Specifically, using the prompt gamma reactions from fast neutrons, the relative concentration of the elements C, N, O in a given spatial cube, called a voxel, can be determined. Using the specific ratios of these three elements in high explosives, the presence and the location of an explosive can be identified.

More particularly, by using suitable gamma detector arrays and by scanning the neutron beam pulse along two axes, a 3D map of the element concentration in the different voxels can in principle be constructed.

The PFNA technique is particularly suitable for examining a small amount of explosives hidden within a large object of relatively low average density.

However, a very short mono-energetic neutron pulse is required if a reasonable spatial resolution is to be achieved. Typically, such neutron source is produced from a linear accelerator. Although such accelerator based PFNA systems have been developed for the examination of cargo containers and for air security applications with good results, the large size and substantial shielding requirement associated with the linear accelerator means that the system cannot be mobile, so that the technique is not appropriate for demining or airport applications.

In addition, all the existing neutron-based detection techniques, while being accurate in terms of analysing the chemical contents of a sample, are slow (it takes typically several minutes to perform a single detection).

This bottleneck is the result of the standard photon counting technique required to create the gamma spectrum build-up and hence to allow the chemical elements in the sample to be detected.

The reason is that the detector can receive only one photon at a time and must wait for a detection time window to analyse the energy of the photon before the next photon can arrive.

With a very high intensity neutron pulse, it is not possible to record each activation gamma photon or backscattered neutron individually, as the phenomenon of pulse piling-up will occur. Indeed, if more than one photon is detected in a given measurement time window, the photons are considered as arriving "together" and therefore are not discriminated from each other.

The maximum rate of single photon detection and analysis, therefore limits the maximum neutron fluence usable in a single pulse.

This leads, as cited above, to a detection time of several minutes to construct useful spectra with a sufficiently high signal to noise ratio for positive detection. The reason is that 10⁶ or more measurements, each typically requiring 1 microsecond or more, are needed to build up a good quality spectrum.

Moreover, the neutron source used in such known systems involves radioactive elements or electrical generators using radioactive targets, which is highly undesirable in particular in civil environments.

The document DE 103 23 093 describes a method for the detection of dangerous goods such as explosives, chemical warfare agents or narcotics in pieces of luggage. The items to be examined are irradiated with high-speed neutrons.

To evaluate said operation, the resultant thermal neutrons are detected using multiple channels, in a time resolved manner, using neutron counting tubes, and their decay curve as a function of time is developed. At the same time, induced gamma radiation due to inelastic scattering and captured radiation is registered, using two or more gamma detectors and gamma spectra construction. The coincidence analysis of the n-detector time decay signal and the gamma spectra from multiple channels is used to provide detection criteria.

Furthermore, the document WO 99/53344 describes a system to detect substances with a pulsed neutron generator. It comprises a detection system with a single gamma detector, which outputs three separate sets of spectrum of the gamma rays, originating from fast neutron inelastic collisions, from thermal neutron reactions and by neutron activations respectively. The three spectra are obtained by sampling the gamma photons resulting after each pulse of interrogating neutrons at different time period after the launch of the neutron pulse, while each of the spectra is established over a period of time by repetitively pulsing the neutron source and measuring the energy of the detected gamma rays in a single photon counting scheme.

The document US 5 446 288 provides a portable system comprising: a weak radioactive source for providing a flux of fast neutrons; a detector for detecting a scattered flux of thermal neutrons and for generating a detector output signal indicating the magnitude of said scattered flux; and an output device, coupled to said detector, for converting said detector output signal to a format indicating the magnitude of said scattered flux to a user.

### Summary of the invention

The present invention seeks to overcome these shortcomings of conventional neutron-base detection techniques for inspecting items.

More particularly, in addition to the self-evident considerations of cost-effectiveness and acceptable footprint, a system offering trustworthiness and very fast detection time would be highly desirable.

It is an object of the present invention to provide a new material detection technology based on impulse neutron interrogation methodology which avoids the conventional single photon counting method to characterize the photons and neutrons, activated and backscattered from an object.

It is a further object of the present invention to provide a versatile detection system covering a wide range of energies, including their evolution in time, with a single measurement.

Another object of the present invention is to provide an identification system allowing to significantly reduce false alarm rates and to provide improved data output for operator decision.

It is also an object of the present invention to provide a system capable of detecting both conventional contrabands like narcotics and certain nuclear materials, which may be hidden in metallic containers that are opaque to conventional X-ray inspection systems.

It is still a further object of the present invention to provide a detection system which overcomes the limitation due to pulse piling-up.

Still another object of the present invention is to provide a detection system allowing to detect the presence of bulk explosives in airport baggage or of landmines in demining by means of a single high intensity neutron pulse and with a short response time.

Accordingly, the present invention provides a detection system according to claim 1.

Preferred but non-limiting aspects of this system are as follows:
* each detector assembly comprises a respective energy band-pass filter.
* each detector assembly comprises a scintillator coupled to a photomultiplier by means of optical fibers.
* each detector assembly is sensitive to gamma photons and neutrons.
* said particles are neutrons and said particle source comprises:
   - first and second electrodes,
   - a plasma ion source at the first electrode,
   - a plasma ion source driver for allowing a ion plasma containing deuterons to develop towards the second electrode,
   - means for applying between said electrodes a short high voltage pulse at a time at which said ion plasma is in a transitional state with a space distribution of deuterons at a distance from said second electrode, so as to accelerate said deuterons towards said second electrode while overcoming the space charge current limit of a conventional vacuum diode, and
   - said second electrode forming a lithium-bearing target, so as to generate said neutrons at said second electrode by deuteron/lithium interaction.
* said neutrons have an energy of at least 3 MeV, which is appropriate for the detection of nuclear materials.
* said neutrons have an energy of at least 5 MeV, which is appropriate for the detection of carbon-based materials, while not interacting with possible surrounding elements.
* said neutrons have an energy of at least 8 MeV, which is appropriate for the detection of explosives, as they interact with all four elements H, C, N, O common to most explosives.
* the signals delivered by the detector assemblies correspond to one single pulse of particles from said source.
* the particle source is also capable of generating gamma photons resulting from neutron interaction within the source itself.

According to a second aspect, the present invention provides a method for detecting properties of materials according to claim 9.

In a preferred manner, said application step comprises the application of a single pulse of a flux of energetic particles.

In such case, said application step may further comprise the application of a secondary pulse, made of different energetic particles and induced from said single pulse.

Preferably, the energetic particles of said single pulse are neutrons, while the energetic particles of said secondary pulse are gamma photons.

### Brief description of the drawings

The present invention will be better understood from the following exemplary description of a preferred embodiment thereof, made with reference to the appended drawings in which:
- Figure 1 is a block diagram of an identification system according to the present invention;
- Figure 2 illustrates in greater detail a detection unit comprised in the system of Figure 1; and
- Figure 3 is a time chart illustrating a typical signal collection in relation with pulse emission and their arrival at the item to be analyzed.

### Detailed description of a preferred embodiment of the invention

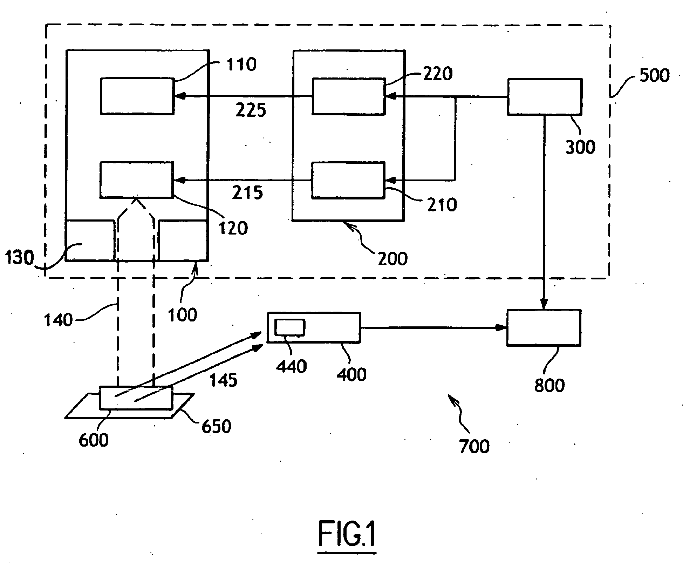

Now referring to the drawings, an identification system 700 according to the present invention is depicted in the figure 1 and comprises:
- a conveyor system 650 for items 600 to be inspected;
- an energetic particle source 500;
- a detection unit 400; and
- a data processing unit 800.

The source 500 generates a high flux and a high intensity of short pulse energetic particles directed toward an item 600 to be checked.

The source includes a particle generator 100 which generates the flux of energetic particles in response to a pulsed power unit 200 controlled by a control unit 300.

In operation, a short pulse of high density, high flux energetic particles is generated and directed towards an item 600 placed in an inspection region towards which a beam collimator 130 is directed.

The conveyor system 650 can be conventional and is used for moving the items 600 such as pieces of baggage by appropriate increments through the inspection region.

The source 500 is used in combination with the detection unit 400 to detect electromagnetic radiation and energetic particles signals representative of the materials contained in the inspected item 600, upon interaction with the penetrating source of energetic particles.

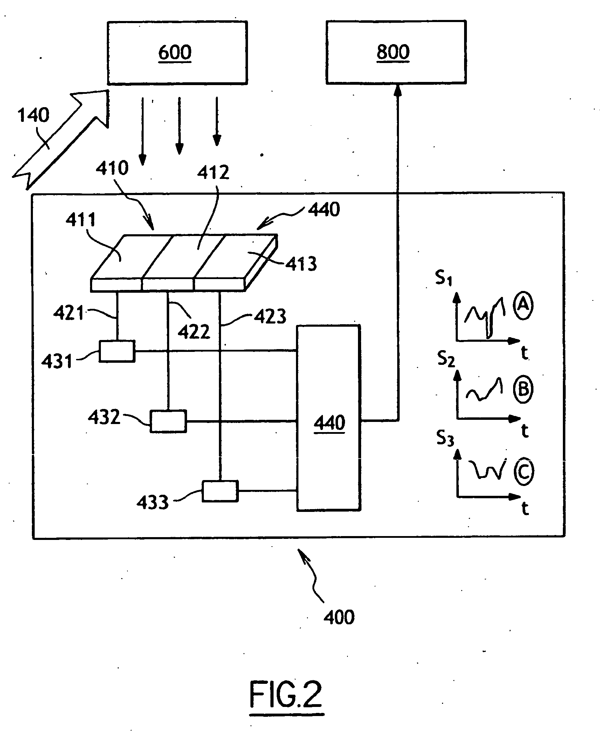

As shown in Figure 2, the detection unit 400 comprises an array of detectors 410 having a wide set of energy responses and capable of detecting both gamma photons and/or neutrons backscattered from item 600 when exposed to the source 500. Each detector 410 is sensitive to a given range of energy for the electromagnetic rays and energetic particles rays backscattered from the object.

The detectors together provide data which can be processed at unit 800 to give a unique signature for specific chemical or nuclear materials or compounds to be looked for.

Correlatively, a detection according to the present invention is based on characteristic gamma signature recognition which can be acquired during a single pulse of energetic particles (although multiple pulse detection is also possible) and does not require detailed energy resolution contrary to prior art systems.

The data processing unit 800 analyzes the signals provided by the detector array in order to generate the gamma signature of item 600. Then a statistical comparison of the calculated signature with a database of reference signatures is used to make the decision on the presence or absence of certain materials or compounds in said item.

A more detailed description of the various components of the system will now be given.

### Particle source 500

The particle generator 100 is driven by a pulsed power supply unit 200 to generate short pulses 140 of energetic particles.

These pulses 140 are generated on demand upon a control trigger delivered by a control unit 300. At all other times, the whole system 500 is in an "off" condition.

The particle generator 100 is contained in a vacuum chamber 150 containing a pair of spaced electrodes, i.e. an emitting electrode 110 and a target electrode 120.

Typically, the distance between the two electrodes 110 and 120 is a few centimetres and the pressure is between 0.1 and 10 Pa.

A high voltage driver 220 for the emitting electrode 110 is provided in the power supply unit 200 and is used to power said electrode by applying a suitable voltage pulse 225 between a pair of electrode members (not shown) belonging to said electrode and forming a plasma discharge ion source.

A low pressure plasma with a plasma density having an order of magnitude of 10¹³ particles/cm³ or more is thus created in the vicinity of electrode 110 and then develops a space distribution of charged particles within chamber 150. After a predetermined time delay dt, a high voltage pulse 215 generated at pulse generator 210 also provided in power supply unit 200 is applied between electrodes 110 and 120 in order to accelerate towards the second electrode 120 the particles having a predetermined charge sign contained in the plasma.

The time delay dt is selected as a function of the voltage level of the plasma triggering pulse 225, the accelerating voltage pulse 215, the geometry of the diode formed by the two electrodes 110 and 120 and the pressure within the chamber 150.

The control unit 300 is capable of triggering driver 220 and then generator 210 according to the above time delay.

The synchronised command control the high voltage pulse supply 210 to start applying a suitable pulse voltage 215, after a time delay dt, between the two electrodes 110 and 120 so that a charged particles beam is extracted from the plasma.

According to an aspect of the invention, the high voltage pulse generator 210 comprises in a manner known per se a voltage multiplication circuit followed by a pulse compression circuit (not shown).

More particularly, a mains voltage source such as 220 V, 50 Hz is first increased to 30 kV using a conventional electronic inverter unit. This voltage is used to feed a 4 stage Marx circuit responsive to a trigger control to produce a voltage pulse of 120 kV. This voltage is then used to charge a pulse shaping circuit to produce a 5 ns pulse of 120 kV. The output of this pulse shaping circuit is coupled to a pulse transformer, providing a final 5 ns voltage pulse 215 of 720 kV.

Upon application of pulse 215, charged particles contained in the plasma are accelerated to form a charged beam with a high current (typically more than 1 kA) which impinges on the electrode 120 serving as target electrode with an energy which can reach 500 keV or more, thereby producing a flux of highly energetic particles as the result of a charged particle-induced nuclear reaction.

It will be appreciated here that the principle of operation of source 500, where a high-energy flux of charged particles is produced by the direct application of a ultra-short high voltage pulse 215 to electrodes between which an ion plasma is in a transitional state, allows to overcome the space charge current limit of a conventional vacuum diode. For instance, a short pulse (<10 ns), high current (> kA), high-energy (> 700 keV) charged particle beam can be generated.

With the particle source as described above, the flux 140 of energetic particles 140 is emitted in an isotropic manner. In order to generate a beam directed towards the item 600 to be analyzed, a suitable collimator 130 is provided.

Finally, it will be noted that the control unit 300 may also serve as a monitoring unit, providing control and status information on all modules of source 500. For this purpose, unit 300 is coupled to a set of safety sensors and/or detectors to ensure safety interlock and proper operation of source 500.

For high speed screening operations, the source 500 can be repeatedly activated, e.g. one to several times per second.

The source 500 may be used for generating various types of energetic particle beams. In a preferred embodiment of the invention, such particles are neutrons, which are generated by the impact of an energetic charged deuteron particle beam of around 10 ns duration at current value having an order of magnitude of kA, on a lithium alloy target electrode 120, thereby producing a 10 ns pulse 140 of more than 10⁸ neutrons, thus providing a high equivalent fluence rate of 10¹⁶ neutrons/second with a broad energy distribution up to 14 MeV.

The source 500 as described in the foregoing allows to detect substantially all gamma photons and neutrons backscattered by the item over a very short duration.

By comparison, a conventional sealed neutron tube typically provides a small quantity of neutrons in 10 microsecond bursts which have to be repeated at a frequency of 1 kHz or more to obtain an equivalent fluency rate of 10⁸ n/s, and several minutes of operation are required to provide sufficient gamma photon data for analysis. This leads to a radiation dose at least 2 orders of magnitudes higher than the one occurring with the present invention.

In a non limiting example, at a distance of 3 m, an item 600 with a 10 cm² exposed area will receive a neutron flux at an equivalent rate of 10¹¹ n/s from the single pulse obtained with the source 500 of the present invention.

### Detection unit 400

As shown in Figure 2, the detection unit 400 comprises a gamma photon detection part 410 comprising an array of detectors (three in the present example) 411, 412, 413.

Each detector is connected to a photomultiplier 431, 432, 433 by means of a respective optical fiber 421, 422, 423.

Each detector preferably comprises a conventional plastic scintillator (e.g. of well known NE1 02A type) of appropriate surface area selected so as to be sensitive both to gamma photons and neutrons, and outputs a signal representing the gamma photons and neutrons backscattered from the item 600 when exposed to the beam 140 generated by source 500.

The size of each scintillator is for instance 180 mm x 180 mm x 25 mm. More generally, a large size of the scintillators allows to substantially improve signal/noise ratio.

Each detector 411, 412 and 413 has a response within a specific energy spectrum, and this is preferably obtained by placing materials (not shown) on the travel path of neutrons and gamma photons from the item 600 to the respective detector, these materials acting as energy band-pass filters in different energy ranges for each detector 411, 412 and 413.

The output amplitude of each detector is therefore related to the spectral content within that range, and detectors 411, 412 and 413 provide respective signals A, B and C indicative of a quantity of received radiation/particles.

It will be appreciated that, by connecting the scintillators to photomultipliers via optical fibres, the photomultipliers can be placed at a distance from the source of radiation and be shielded against the effect of energetic particles and electromagnetic radiation generating noise in said photomultipliers.

The photomultipliers 431, 432 and 433 operate in current detection mode to allow real time measurement of the evolution of the photons/particles impinging on the respective scintillators.

In this regard, instead of measuring the energy of individual gamma photons or neutrons, the current signal produced by the collection of particles and photons arriving at a given detector 411, 412 or 413 is recorded as a function of time.

Therefore, the conventional issue of pulse pile-up when particles impinge on the scintillator within a very short time interval, resulting in counting errors, is avoided.

The signal outputs from the photomultipliers 431, 432 and 433 are supplied to analog-to-digital converters globally referenced 440 so as to provide a corresponding digital data stream representative of the photons/particles received from irradiated item 600 as a function of time in different energy bands.

As an example, a 4-channel Tektronix TDS3034 transient digitizer with a maximum sampling rate of 2.5GS/s and a maximum data memory depth of 10 k samples can be used.

Preferably, the digitalized data are recorded for e.g. 20 µs after each particle pulse 140 is applied to the item, with a sampling rate of 2 ns at most so as to keep within the memory depth of the digitizer.

The signals provided by the detection unit 400 combine the results from any neutron interaction modes in the materials of item 600, including elastic, inelastic and captured reactions, that occur in response to the single ultra-short high energy neutron pulse 140 and that lead to the emission of gamma photons or neutrons.

More particularly, the detection unit 400 detects prompt and delayed gamma photons generated from fast and thermal neutrons, as well as neutrons backscattered or emitted from the sample, during different periods after the single short neutron pulse is triggered.

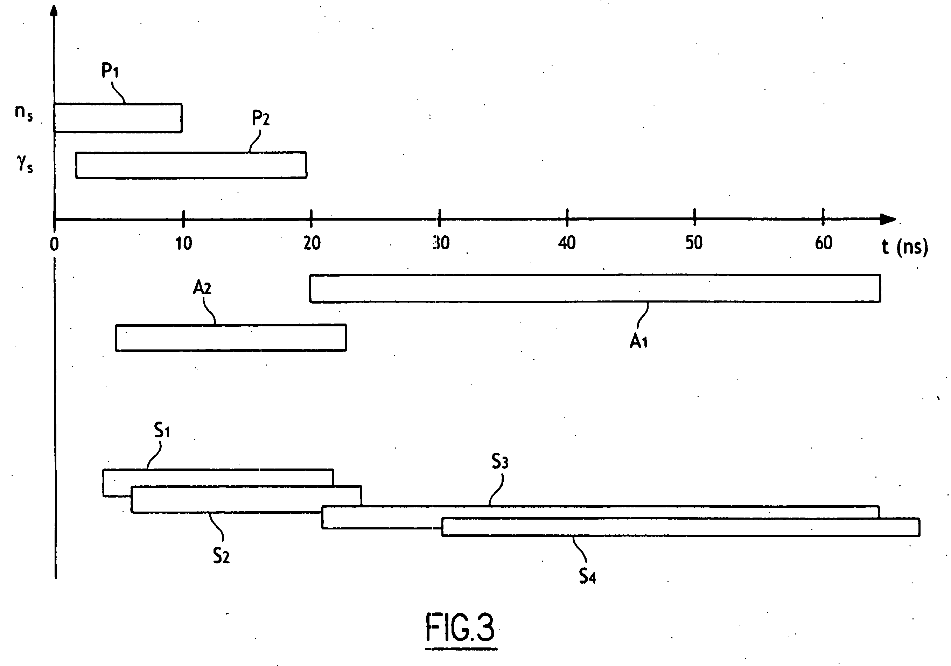

An example of a typical timing of signal detection in relation with neutron pulse generation is given in figure 3.

In this example, a single pulse P1 of about 10⁸ neutrons having a duration of 10 ns is emitted in a 4π solid angle. A secondary pulse of gamma photons P2 is also emitted with a slight time shift and a slightly longer duration. This gamma pulse P2 is generated as a result of the interaction of the neutron pulse P1 with the immediate surrounding of the neutron-generating target, as well as within the target itself.

The blocks A1 and A2 correspond respectively to the neutrons of pulse P1 arriving on a target located 1 metre away from the source, and to the gamma photons of pulse P2 arriving on said target.

The detected signals as shown in the bottom of Figure 3 are as follows:
- initially, the collected signals S1 are due to a fraction of the gamma pulse P2 arriving directly at the detectors;
- this is closely followed by the signals S2 which originate from a certain quantity of the gamma pulse P2 being backscattered from item 600;
- some few tens of nanoseconds later, the received signals S3 are composed of high-energy gamma photons produced when high energy neutrons travel directly through item 600 and have non-elastic collisions with the nuclei of the item material(s); the very short neutron pulse P1 and the relatively slow speed of travel (a 10 MeV neutron travels about 4.4 cm in one nanosecond) allow good spatial discrimination; in addition, the high intensity of the neutron flux leads to a good signal to noise ratio from the detectors;
- the latter part of signals S3 are gamma photons generated from the interaction with item 600 of a large quantity of delayed neutrons resulting from the scattering of the uncollimated neutrons at the source;
- these signals are followed (time scale of the order of the microsecond) by signals S4 corresponding to neutrons backscattered from the item;
- the last detected signals (after ten microseconds or more, not shown) result from captured gamma photons produced from neutrons which have been "thermalized" in the source collimator, as well as from the sample and its surrounding. These thermal neutrons are captured by the nuclei of the item material(s) which in turn generate gamma photons.

Many of these interactions are applicable for a wide variety of elements/compounds. Therefore, by recording the detected signals over a long period of time (typically tens of microseconds) after the start of the neutron pulse 140, very meaningful signals are obtained, esp. when considering evolution over time.

Preferably, a gain adjustment circuit is incorporated into each of the detector assemblies (scintillator + optical fibres + photomultiplier) in order to compensate for the variations in the coupling efficiency between the scintillator and an optical fiber.

Moreover, the detector assemblies are advantageously cross-calibrated by taking a set of measurements without the energy filters in front of the detectors and then with identical energy filters on all detectors.

All detection units are calibrated in the same way and the gain of each unit is adjusted so that the variation in signal output between all units is within a given range (e.g. a factor of two at most).

The calibration can be made with a reference pulsed light source coupled to a plurality of fibre optics each coupled to a respective photomultiplier. In this way, each photomultiplier will be illuminated by the same calibration light source through identical fibre optics coupling.

An automatic calibration process can also be conducted with one or several samples of well defined materials, for example organic materials such as Melamine and Polythene. Such process allows to compensate for possible drifts in the detectors sensitivity.

### Data Processing unit 800

The data processing unit 800 comprises suitable signal processing power for the plurality of signals A, B and C received from detection unit 400.

Preferably, such processing involves applying a predetermined set of algorithms to said signals, including their evolution over time, in order to generate a signature associated with each item 600 analyzed.

Once an item has been analyzed, its signature is applied to a database for comparison with a set of reference signatures corresponding to different known materials or compounds or substances, so that the presence of any such materials or compounds or substances can be quickly identified and possibly quantified.

Preferably, unit 800 is programmed so as to provide an operator with simple yes/no answers for different types of materials or compounds or substances in a very short response time (typical processing times will be from a fraction of second to several seconds with state of the art processing capability.

While the present invention has been described with respect to exemplary embodiments thereof, it will be understood by the skilled person that many variations and modifications can be made thereto.

As indicated in the foregoing, although typical applications of the present invention are security checks for airport baggage and detection of buried objects such as landmines and antipersonnel mines, the present invention may have numerous other applications.

## Claims

1. A detection system, comprising:
- a particle source (500) for generating a pulsed flux (140) of energetic particles and for directing said flux towards an item (600) to be analyzed, said particles being intended to react with nuclei of material(s) In said item,
- a detection unit (400) comprising at least three detector assemblies (411, 421, 431; 412, 422, 432; 413, 423, 433), and
- a data processing unit (800) connected to the outputs of said detectors, capable of generating a signature from said signals following the application of said pulsed flux to said item, including time-related signal features, and for comparing said signature with stored reference signatures,
**characterized in that**:
- the energetic particles include both neutrons and gamma photons, and
- the detector assemblies are responsive to neutrons and gamma photons coming from said item and impinging thereon in response to said flux of energetic particles, each being sensitive to a different range of energy, and are arranged to operate in current detection mode to deliver current signals representative of impinging gamma photons and neutrons over time.

2. The system of claim 1, wherein each detector assembly comprises a respective energy band-pass filter, and each band-pass filter possesses a response in a different energy spectrum.

3. The system of claim 1 or 2, wherein each detector assembly comprises a scintillator (411; 412; 413) coupled to a photomultiplier (431; 432; 433) by means of optical fibres (421; 422; 423).

4. The system of any one of claims 1-3, wherein said particle source comprises:
- first and second electrodes (110, 120),
- a plasma ion source at the first electrode,
- a plasma ion source driver (220) for allowing a ion plasma containing deuterons to develop towards the second electrode,
- means (210) for applying between said electrodes a short high voltage pulse at a time at which said ion plasma is in a transitional state with a space distribution of deuterons at a distance from said second electrode, so as to accelerate said deuterons towards said second electrode while overcoming the space charge current limit of a conventional vacuum diode,
- said second electrode (120) forming a lithium-bearing target, so as to generate said neutrons at said second electrode by deuteron/lithium interaction, and
- said neutrons interacting with said target to produce gamma photons.

5. The system of any one of claims 1-4, wherein said neutrons have an energy of at least 3 MeV.

6. The system of any one of claims 1-5, wherein said neutrons have an energy of at least 5 MeV.

7. The system of any one of claims 1-6, wherein said neutrons have an energy of at least 8 MeV.

8. The system of any one of claims 1-7, wherein the signals delivered by the detector assemblies correspond to one single pulse of particles from said source.

9. A method for detecting properties of materials, substances or compounds contained in items, wherein a pulsed flux of energetic particles is applied to an item to be analyzed, said particles being intended to react with nuclei of material(s) in said item,
**characterized in that** the energetic particles include both neutrons and gamma photons, and the method further comprises the following steps:
- detecting neutrons and gamma photons coming from said item in response to said application in current detection mode in at least three energy ranges wherein each energy range is different from the others, so as to deliver current signals representative of impinging neutrons and gamma photons over time,
- delivering time resolved signals representative of neutrons and gamma photons thus detected,
- generating a signature from said signals, including from time-correlated features of said signals, and
- comparing said signature with stored reference signatures.

10. The method of claim 9, wherein said particles are neutrons with a energy of at least 3 MeV.

11. The method of claim 9 or 10, wherein said particles are neutrons with a energy of at least 5 MeV.

12. The method of any one of claims 9-11, wherein said particles are neutrons with a energy of at least 8 MeV.

## Patentansprüche

1. Detektionssystem, umfassend:
- eine Teilchenquelle (500) zum Erzeugen eines gepulsten Flusses (140) energiegeladener Teilchen und zum Lenken des Flusses in Richtung eines zu analysierenden Gegenstandes (600), wobei die Teilchen dafür vorgesehen sind, mit Kernen von einem oder mehreren Materialien in dem Gegenstand zu reagieren,
- eine Detektionseinheit (400), die mindestens drei Detektorbaugruppen (411, 421, 431; 412, 422, 432; 413, 423, 433) umfasst, und
- eine Datenverarbeitungseinheit (800), die mit den Ausgängen der Detektoren verbunden ist und die in der Lage ist, eine Signatur aus den Signalen nach dem Einwirken des gepulsten Flusses auf den Gegenstand zu erzeugen, einschließlich zeitbezogener Signalmerkmale, und zum Vergleichen der Signatur mit gespeicherten Referenzsignaturen,
**dadurch gekennzeichnet, dass**:
- die energiegeladenen Teilchen sowohl Neutronen als auch Gammaphotonen enthalten, und
- die Detektorbaugruppen auf Neutronen und Gammaphotonen ansprechen, die als Ergebnis des Flusses energiegeladener Teilchen von dem Gegenstand kommen und auf die Detektorbaugruppen auftreffen, wobei jede Detektorbaugruppe auf einen anderen Energiebereich anspricht, und wobei die Detektorbaugruppen dafür ausgelegt sind, in einem Stromdetektionsmodus zu arbeiten, um Stromsignale auszugeben, die für auftreffende Gammaphotonen und Neutronen im zeitlichen Verlauf repräsentativ sind.

2. System nach Anspruch 1, wobei jede Detektorbaugruppe ein jeweiliges Energiebandpassfilter umfasst und jedes Bandpassfilter auf ein anderes Energiespektrum anspricht.

3. System nach Anspruch 1 oder 2, wobei jede Detektorbaugruppe einen Szintillator (411; 412; 413) umfasst, der über optische Fasern (421; 422; 423) mit einem Photoelektronenvervielfacher (431; 432; 433) gekoppelt ist.

4. System nach einem der Ansprüche 1-3, wobei die Teilchenquelle umfasst:
- eine erste und eine zweite Elektrode (110, 120),
- eine Plasma-Ionenquelle an der ersten Elektrode,
- einen Plasma-Ionenquellentreiber (220), um es zu ermöglichen, dass sich ein Deuteronen enthaltendes Ionenplasma in Richtung der zweiten Elektrode entwickelt,
- Mittel (210) zum Anlegen - zwischen den Elektroden - eines kurzen Hochspannungsimpulses zu einem Zeitpunkt, an dem sich das Ionenplasma in einem Übergangszustand - mit einer Raumverteilung von Deuteronen in einer Entfernung von der zweiten Elektrode - befindet, so dass die Deuteronen in Richtung der zweiten Elektrode beschleunigt werden, während die Raumladungsstromgrenze einer herkömmlichen Vakuumdiode überwunden wird,
- wobei die zweite Elektrode (120) ein Lithium-tragendes Ziel bildet, so dass die Neutronen an der zweiten Elektrode durch eine Wechselwirkung zwischen Deuteronen und Lithium erzeugt werden, und
- wobei die Neutronen mit dem Ziel in Wechselwirkung treten, um Gammaphotonen zu erzeugen.

5. System nach einem der Ansprüche 1-4, wobei die Neutronen eine Energie von mindestens 3 MeV haben.

6. System nach einem der Ansprüche 1-5, wobei die Neutronen eine Energie von mindestens 5 MeV haben.

7. System nach einem der Ansprüche 1-6, wobei die Neutronen eine Energie von mindestens 8 MeV haben.

8. System nach einem der Ansprüche 1-7, wobei die von den Detektorbaugruppen ausgegebenen Signale einem einzelnen Teilchenimpuls von der Quelle entsprechen.

9. Verfahren zum Detektieren von Eigenschaften von Materialien, Stoffen oder Verbindungen, die in Gegenständen enthalten sind, wobei ein gepulster Fluss energiegeladener Teilchen auf einen zu analysierenden Gegenstand einwirkt, wobei die Teilchen dafür vorgesehen sind, mit Kernen von einem oder mehreren Materialien in dem Gegenstand zu reagieren, **dadurch gekennzeichnet, dass** die energiegeladenen Teilchen sowohl Neutronen als auch Gammaphotonen umfassen, und dass das Verfahren des Weiteren die folgenden Schritte umfasst:
- Detektieren von Neutronen und Gammaphotonen, die als Ergebnis des Einwirkens von dem Gegenstand kommen, in einem Stromdetektionsmodus in mindestens drei Energiebereichen, wobei jeder Energiebereich von den anderen Energiebereichen verschieden ist, um Stromsignale auszugeben, die für auftreffende Neutronen und Gammaphotonen im zeitlichen Verlauf repräsentativ sind,
- Ausgeben zeitlich aufgelöster Signale, die für auf diese Weise detektierte Neutronen und Gammaphotonen repräsentativ sind,
- Erzeugen einer Signatur aus den Signalen, einschließlich aus zeitkorrelierten Merkmalen der Signale, und
- Vergleichen der Signatur mit gespeicherten Referenzsignaturen.

10. Verfahren nach Anspruch 9, wobei die Teilchen Neutronen mit einer Energie von mindestens 3 MeV sind.

11. Verfahren nach Anspruch 9 oder 10, wobei die Teilchen Neutronen mit einer Energie von mindestens 5 MeV sind.

12. Verfahren nach einem der Ansprüche 9-11, wobei die Teilchen Neutronen mit einer Energie von mindestens 8 MeV sind.

## Revendications

1. Système de détection, comprenant :
- une source de particules (500) pour générer un flux pulsé (140) de particules d'énergie et pour diriger ledit flux vers un élément (600) à analyser, lesdites particules étant prévues pour réagir avec le(s) noyau (x) de matériau (x) dans l'élément ;
- un module de détection (400) comprenant au moins trois ensembles de détecteurs (411, 421, 431 ; 412, 422, 432 ; 413, 423, 433) ; et
- un module de traitement de données (800) connecté aux sorties desdits détecteurs, apte à générer une signature à partir desdits signaux consécutivement à l'application dudit flux pulsé sur l'élément, comprenant des caractéristiques de signal lié au temps, et apte à comparer ladite signature à des signatures de référence enregistrées ;
**caractérisé en ce que** :
- les particules d'énergie comprennent à la fois des neutrons et des photons gamma ; et
- les ensembles de détecteurs sont sensibles aux neutrons et aux photons gamma qui arrivent dudit élément et viennent les impacter en réponse au dit flux de particules d'énergie, chacun étant sensible à une plage d'énergie différente, les ensembles de détecteurs étant en outre configurés de façon à opérer dans un mode de détection de courant de sorte à délivrer des signaux de courant représentatifs des photons gamma et des neutrons impactés sur la durée.

2. Système selon la revendication 1, dans lequel chaque ensemble détecteur comprend un filtre passe-bande d'énergie respective, et chaque filtre passe-bande possède une réponse dans un spectre d'énergie différent.

3. Système selon la revendication 1 ou 2, dans lequel chaque ensemble détecteur comprend un scintillateur (411 ; 412 ; 413) couplé à un photomultiplicateur (431 ; 432 ; 433) au moyen de fibres optiques (421 ; 422 ; 423).

4. Système selon l'une quelconque des revendications 1 à 3, dans lequel ladite source de particules comprend :
- des première et seconde électrodes (110, 120) ;
- une source de plasma ionisé à la première électrode ;
- un circuit de commande de source d'ions dans le plasma (220) pour permettre à un plasma ionisé contenant des deutérons de se développer vers la seconde électrode ;
- des moyens (210) pour appliquer, entre lesdites électrodes, une courte impulsion de haute tension à un moment où ledit plasma ionisé est dans un état de transition avec une distribution spatiale de deutérons à distance de ladite seconde électrode, afin d'accélérer lesdits deutérons vers la seconde électrode tout en surmontant la limite de courant de charge spatiale d'une diode à vide conventionnelle ;
- ladite seconde électrode (120) formant une cible comprenant du lithium, de manière à générer lesdits neutrons à ladite seconde électrode via une interaction deutérons/lithium ; et
- lesdits neutrons interagissant avec la cible pour produire des photons gamma.

5. Système selon l'une quelconque des revendications 1 à 4, dans lequel lesdits neutrons ont une énergie d'au moins 3 MeV.

6. Système selon l'une quelconque des revendications 1 à 5, dans lequel lesdits neutrons ont une énergie d'au moins 5 MeV.

7. Système selon l'une quelconque des revendications 1 à 6, dans lequel lesdits neutrons ont une énergie d'au moins 8 MeV.

8. Système selon l'une quelconque des revendications 1 à 7, dans lequel les signaux délivrés par les ensembles détecteurs correspondent à une seule impulsion de particules émise par la source.

9. Procédé pour la détection de propriétés de matériaux, substances ou composés contenus dans des éléments, dans lequel un flux pulsé de particules d'énergie est appliqué sur un élément à analyser, lesdites particules étant prévues pour réagir avec le(s) noyau (x) de matériau (x) dans l'élément ;
**caractérisé en ce que** les particules d'énergie comprennent à la fois des neutrons et des photons gamma ; et le procédé comprend par ailleurs les étapes suivantes :
- détecter des neutrons et des photons gamma qui arrivent de l'élément en réponse à l'application dans un mode de détection de courant dans au moins trois plages d'énergie, chaque plage d'énergie étant différente des autres de manière à délivrer des signaux de courant représentatifs des neutrons et des photons gamma qui viennent frapper les détecteurs sur la durée ;
- délivrer des signaux à résolution temporelle représentatifs des neutrons et des photons gamma ainsi détectés ;
- générer une signature à partir desdits signaux, comprenant des caractéristiques corrélées avec le temps desdits signaux ; et
- comparer ladite signature à des signatures de référence enregistrées.

10. Procédé selon la revendication 9, dans lequel lesdites particules sont des neutrons avec une énergie d'au moins 3 MeV.

11. Procédé selon la revendication 9 ou 10, dans lequel lesdites particules sont des neutrons avec une énergie d'au moins 5 MeV.

12. Procédé selon l'une quelconque des revendications 9 à 11, dans lequel lesdites particules sont des neutrons avec une énergie d'au moins 8 MeV.
